# EUROPEAN PATENT APPLICATION

(11) **EP 0 568 344 A1**
(43) Date of publication of application: **03.11.1993**
(21) Application number: 93303320.1
(22) Date of filing: 28.04.1993
(51) Int. Cl.: C07D 493/10, B41M 5/145, B41M 5/30

(54) **Fluoran compounds and process for their production**

(30) Priority: 28.04.1992 JP 109830/92; 02.11.1992 JP 294617/92
(71) Applicant: FUJI PHOTO FILM CO., LTD., Kanagawa (JP)
(72) Inventor: Takeda, Akihiko, c/p Fuji Photo Film Co.,Ltd., Fujinomiya-shi, Shizuoka (JP); Satomura, Masato, c/o Fuji Photo Film Co.,Ltd., Fujinomiya-shi, Shizuoka (JP); Yanagihara, Naoto, c/o Fuji Photo Film Co.,Ltd., Fujinomiya-shi, Shizuoka (JP)
(74) Representative: Moore, Anthony John (GB)

(57) **Abstract**

A fluoran compound of the general formula (I) is useful as a cyan color-former in heat- or pressure-sensitive copying material: 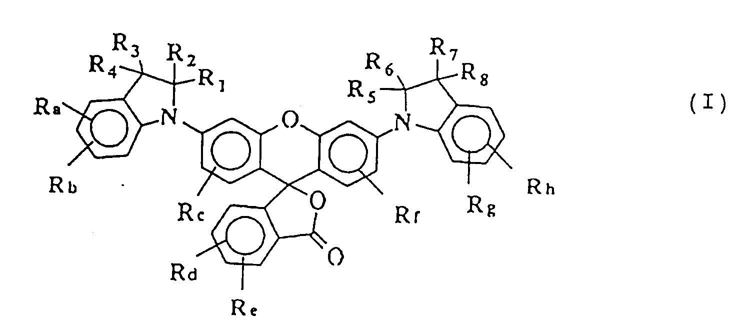
wherein R₁, R₂, R₃, R₄, R₅, R₆, R₇ and R₈ are each hydrogen, substituted or unsubstituted alkyl, aryl or alkoxy, or halogen, R₁ to R₄ and/or R₅ to R₈ may be combined to form a ring, and Rₐ, R_{b}, R_{c}, R_{d}, Rₑ, R_{f}, R_{g} and Rₕ are each hydrogen, substituted or unsubstituted alkyl, alkenyl, amino, carbamoyl, acyl, thioalkoxy or aryloxy, nitro or halogen. The fluoran compounds (I) may be produced by reacting fluorescein chloride and/or fluoroscein bromide with an indoline derivative in the presence of a solvent and zinc chloride and/or aluminum chloride.

## Description

This invention relates to a class of fluoran compounds and a process for their production. More particularly, it relates to a class of fluoran compounds which is useful as an electron-donating compound in a recording material using an electron donating colorless dye and an electron-accepting compound, for example heat sensitive recording material and pressure sensitive recording material.

Various kinds of fluoran compounds are known as electron-donating compounds useful for heat sensitive recording materials and pressure sensitive recording materials, as described in JP-A-62-60694, JP-A-63-924889, JP-A-2-141279, JP-A-2-107476, JP-A-2-209293, JP-A-2-223474, JP-A-2-225082 and JP-A-3-72358 and U.S. Patents 4,929,531 and 4,935,329 (the term "JP-A" as used herein means an "unexamined published Japanese patent application").

Also, processes of producing fluoran compound other than the processes disclosed in the above publications are known. For example, a process of producing a fluoran compound developable to cyan color, comprising reacting fluorescein chloride with an aromatic amine derivative such as diphenyl amine, indole, etc. is known as described in Jikken Kagaku Koza, Vol.5 (Part I), page 1053. The process, however, is impractical from the view points of too high reaction temperature and difficulty in isolating the product due to formation of too large a quantity of by-products.

Accordingly, it is an object of the present invention to provide a fluoran compound which is developable to cyan color by contact with an electron-donating compound and has low tendency to fog.

It is another object of the present invention to provide a process of producing the fluoran compound in a high yield and allowing it to be isolated with ease.

These and other objects and advantages of the present invention will become more apparent from the following detailed description and examples.

The first object of the present invention is accomplished by a fluoran compound represented by formula (I): 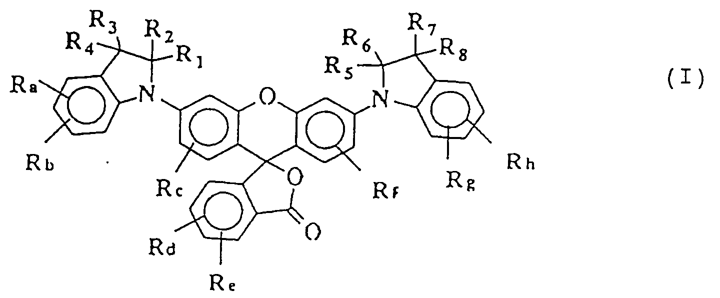
wherein R₁, R₂, R₃, R₄, R₅, R₆, R₇ and R₈ each represent a hydrogen atom, a substituted or unsubstituted alkyl group, a substituted or unsubstituted aryl group, a substituted or unsubstituted alkoxy group or halogen atom, wherein R₁ to R₄ and/or R₅ to R₈ may be combined to form a ring, and Rₐ, R_{b}, R_{c}, R_{d}, Rₑ, R_{f}, R_{g} and Rₕ each represent a hydrogen atom, a substituted or unsubstituted alkyl group, a substituted or unsubstituted aryl group, a substituted or unsubstituted alkoxy group, a substituted or unsubstituted alkenyl group, a halogen atom, a substituted amino group, a substituted carbamoyl group, an acyl group, a thioalkoxy group, an aryloxy group or nitro group.

The second object of the present invention is accomplished by a process of reacting fluorescein chloride and/or fluoroscein bromide with an indoline derivative in the presence of a solvent and zinc chloride and/or aluminum chloride to give a high yield of fluoran compound and to enable the fluoran compound to be isolated with ease.

Preferred groups represented by R₁ to R₈ in formula (I) include a methyl group, ethyl group, propyl group, isopropyl group, butyl group, isobutyl group, pentyl group, isopentyl group, phenyl group, benzyl group, methoxy group, ethoxy group, propoxy group, chlorine atom, bromine atom and fluorine atom. These groups each may further be substituted with an alkyl group, halogen atom, carboxyl group, hydroxy group, alkoxy group, amino group, phenyl group, or benzyl group, or 2-ethylhexyl, n-octyl, dodecyl, pentadecyl, t-decyl, p-t-amylphenyl, p-cumylphenyl, p-phenoxyphenyl, p-dodecylphenyl, 2,4-dibutoxyphenyl, or 2,4-di-t-amylphenyl.

Among the groups represented by R₁ to R₈ in formula (I), the most preferred groups include a hydrogen atom, methyl group, ethyl group, methoxy group, ethoxy group, isopropyl group, chlorine atom and phenyl group.

Preferred groups represented by R₂ to Rₕ in formula (I) include a methyl group, ethyl group, propyl group, isopropyl group, butyl group, isobutyl group, pentyl group, isopentyl group, phenyl group, benzyl group, methoxy group, ethoxy group, propoxy group, vinyl group, allyl group, propenyl group, chlorine atom, bromine atom, fluorine atom, diethylamino group, ethyl amino group, anilino group, benzoylamino group, thiomethoxy group, thiophenoxy group, phenoxy group, p-methoxyphenoxy group or p-phenoxyphenoxy group. These groups each may further be substituted with an alkyl group, halogen atom, carboxyl group, hydroxy group, alkoxy group, amino group, phenyl group and benzyl group. Rₐ and R_{b}, and R_{g} and Rₕ may be combined to form a ring.

Among the groups represented by Rₐ to Rₕ in formula (I), most preferred groups include a hydrogen atom, methyl group, ethyl group, methoxy group, ethoxy group, allyl group, phenyl group and chlorine atom.

Preferably, in formula (I) of the present invention, the groups of R₁ and R₂, R₃ and R₄ are the same as those of R₅ and R₆, and R₇ and R₈, respectively and, further, the groups of Rₐ and R_{b} are the same as those of R_{g} and Rₕ, and the substituted position of Rₐ and R_{b} is the same as that of R_{g} and Rₕ in each indoline ring. In other words, the preferable fluoran compound of formula (I) has the same substituted indoline ring moieties therein.

Among the indoline derivatives to be used in the process of the present invention, more preferred compounds are those represented by formula (II) shown below: 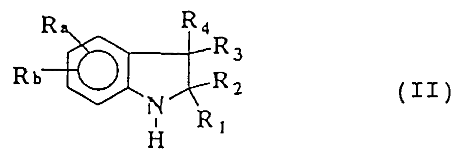
wherein R₁, R₂, R₃ and R₄ each represent a hydrogen atom, a substituted or unsubstituted alkyl group, a substituted or unsubstituted aryl group, a substituted or unsubstituted alkoxy group or halogen atom, wherein R₁ to R₄ and/or Rₐ to R_{b} may be combined to form a 5- to 12-membered ring comprising a non-metallic atom, and Rₐ and R_{b} are each as defined in formula (I).

Examples of the groups represented by each of R₁ to R₄, Rₐ and R_{b} in formula (II) are the same as the groups represented by R₁ to R₈ and Rₐ to Rₕ in formula (I).

Typical examples of the indoline derivative are shown below. They are, however, by no means limitative of the scope of the invention.

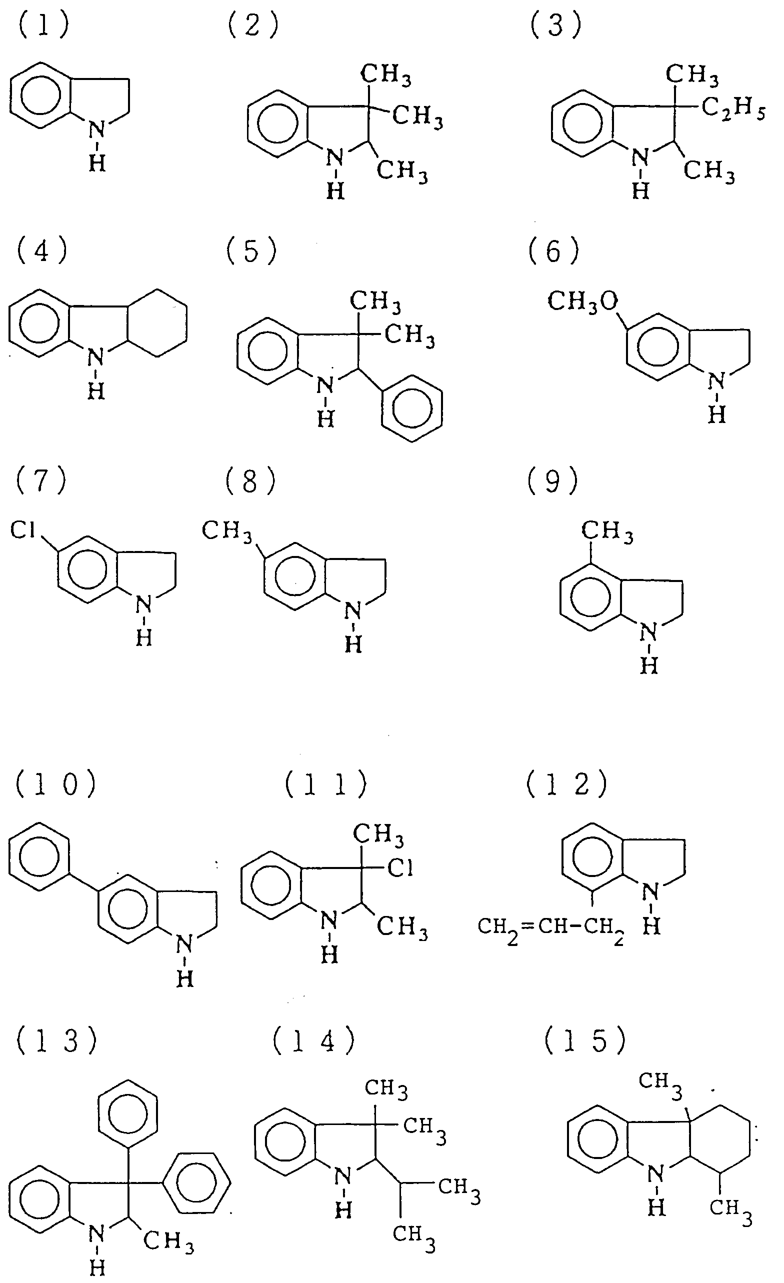

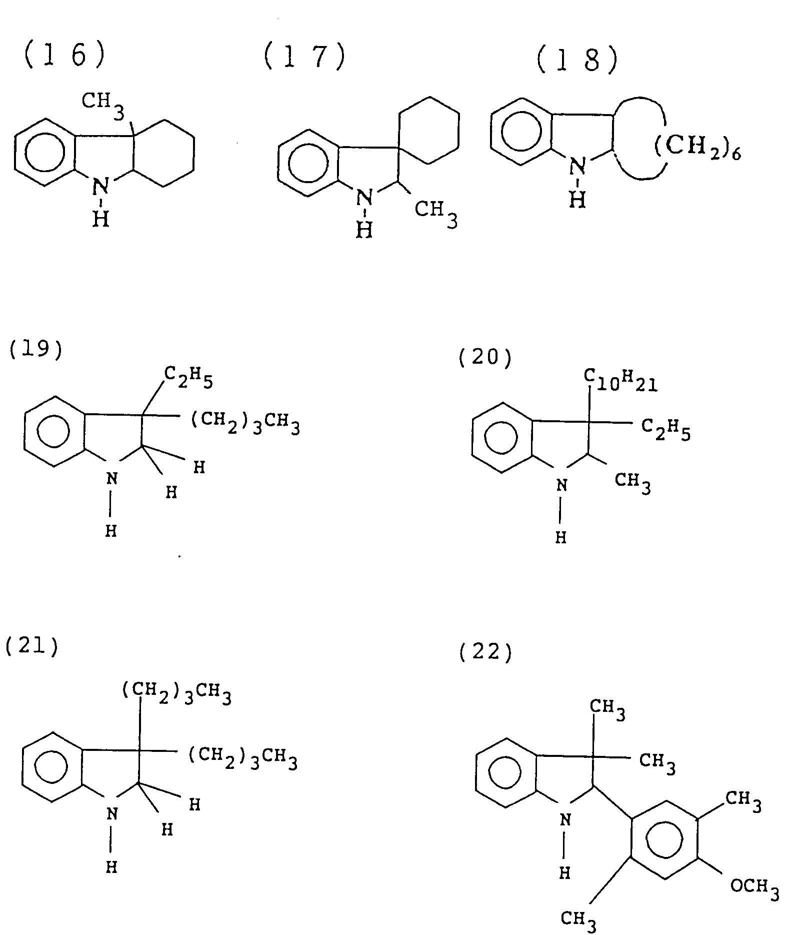

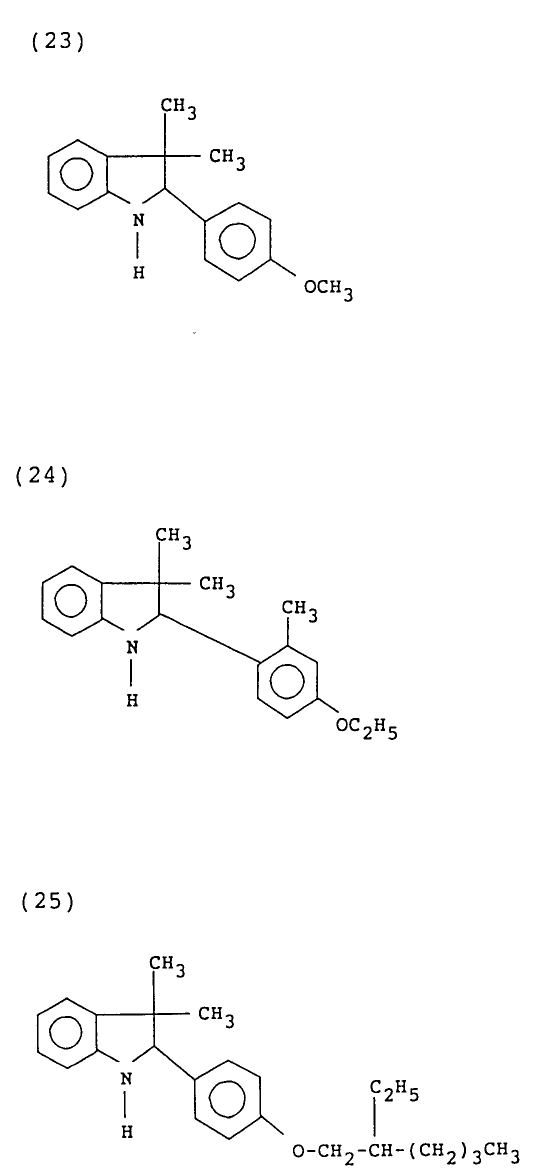

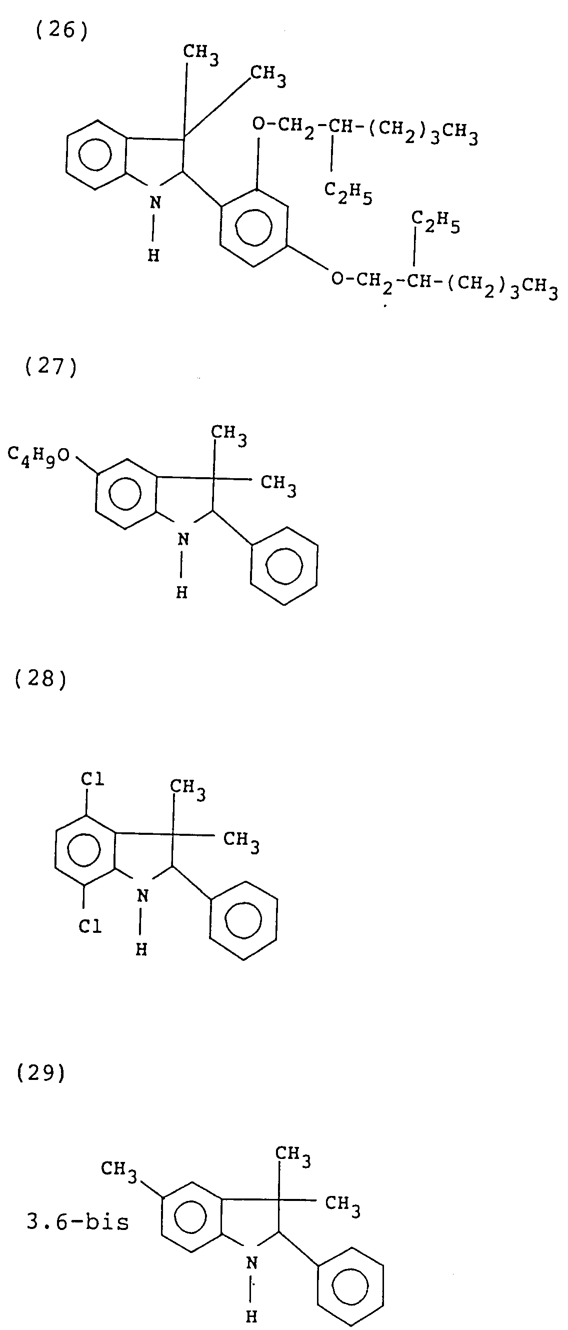

In the practice of the invention, the molar ratio between the fluorescein chloride or fluorescein bromide and indoline derivative is preferably within the range of fluorescein chloride or fluorescein bromide/indoline derivative = 1/1.5 to 1/5, and more preferably within the range of 1/2 to 1/3. The molar ratio between zinc chloride and/or aluminum chloride, and fluorescein chloride and/or fluorescein bromide is preferably within the range of zinc chloride and/or aluminum chloride/fluorescein chloride and/or fluorescein bromide = 0.1/1 to 1/5.

Suitable solvents used for the reaction in the practice of the invention preferably include those having a boiling point of 150°C or more. Specific examples thoereof include an aprotic solvent, such as dimethylsulfoxide, sulfolane, N,N-dimethylacetoamide and N,N-dimethylformamide, a halide solvent such as chlorobenzene and o-, m- and p-dichlorobenzene, nitrobenzene, hexamethylphosphoramide and mixtures thereof. The amount of the solvent to be used in the process in accordance with the present invention is preferably from 30 to 80% based on the solid contained in the reaction system. The reaction temperature is preferably from 100 to 250°C and more preferably from 130 to 180°C.

In another process to produce the fluoran compound of the present invention, the fluoran compound may be obtained by a process comprising reacting a 2-hydroxybenzoylbenzoic acid derivative represented by formula (III) shown below with an indoline derivative represented by formula (IV) also shown below. 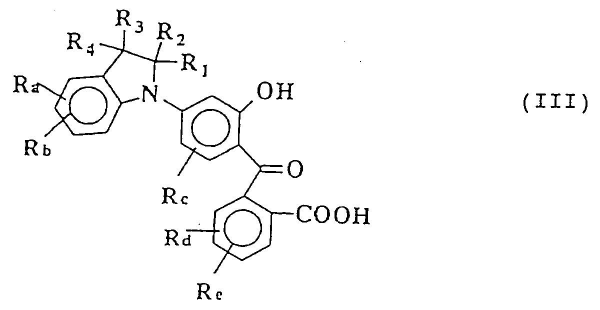
wherein R₁, R₂, R₃ and R₄ each represent a hydrogen atom, a substituted or unsubstituted alkyl group, a substituted or unsubstituted aryl group, a substituted or unsubstituted alkoxy group or halogen atom, wherein R₁ to R₄ and/or Rₐ to R_{b} may be combined to form a ring, and Rₐ, R_{b}, R_{c}, R_{d} and Rₑ each represent hydrogen atom, a substituted or unsubstituted alkyl group, a substituted or unsubstituted aryl group, a substituted or unsubstituted alkoxy group, a substituted or unsubstituted alkenyl group, halogen atom, a substituted amino group, a substituted carbamoyl group, an acyl group, a thioalkoxy group, an aryloxy group or nitro group. 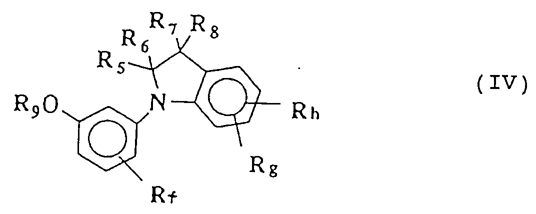
wherein R₅, R₆, R₇ and R₈ each represent a hydrogen atom, a substituted or unsubstituted alkyl group, a substituted or unsubstituted aryl group, a substituted or unsubstituted alkoxy group or halogen atom, wherein R₅ to R₈ and/or R_{g} to Rₕ may be combined to form a ring, and R_{f}, R_{g} and Rₕ each represent a hydrogen atom, a substituted or unsubstituted alkyl group, a substituted or unsubstituted aryl group, a substituted or unsubstituted alkoxy group, an alkenyl group, halogen atom, a substituted amino group, a substituted carbamoyl group, an acyl group, a thioalkoxy group, an aryloxy group or nitro group, and R₉ represents a lower alkyl group.

Preferable and more preferable groups represented by R₁ to R₄ in formula (III) and R₅ to R₈ in formula (IV) include the same as those of R₁ to R₈ in formula (I).

Preferable and more preferable groups represented by Rₐ to Rₑ in formula (III) and R_{f} to Rₕ in formula (IV) include the same as those of Rₐ to Rₕ in formula (I).

Preferable groups represented by R₉ include a methyl group, ethyl group, propyl group and butyl group.

In the practice of the invention by reaction of 2-hydroxy benzoylbenzoic acid derivative of formula (III) and indoline derivative (IV), the molar equivalent ratio of 2-hydroxybenzoylbenzoic acid derivative/indoline derivative is preferably within a range of 1/1 to 1/1.2, and these are reacted in the presence of acidic solvent.

The reaction is preferably conducted at a temperature of from -10 to 50°C for from 2 to 8 hours. When sulfuric acid is used as an acidic solvent, the solvent is preferably used in an amount of 60% or more and more preferably from 80 to 100% based on the solid in the reaction system. Higher acidic concentration is effective to keep the reaction system in liquid state. Sulfuric acid is the most preferable condensing solvent in view of low cost, processability at a low temperature and non-volatility. Sulfuric acid solvent may be used in a combination with fuming sulfuric acid, phosphoric acid anhydride or polyphosphoric acid and a Lewis acid such as zinc chloride or phosphorus oxychloride.

Preferable recrystallization solvents used in the present invention include an aromatic hydrocarbon such as benzene and toluene, a hydrocarbon halide such as monochlorobenzene, dichlorobenzene and chloroform, and an alcohol such as methanol, ethanol and propanol.

Preferred examples of the fluoran compound of formula (I) of the present invention are shown below. They are, however, by no means limitative of the scope of the invention.

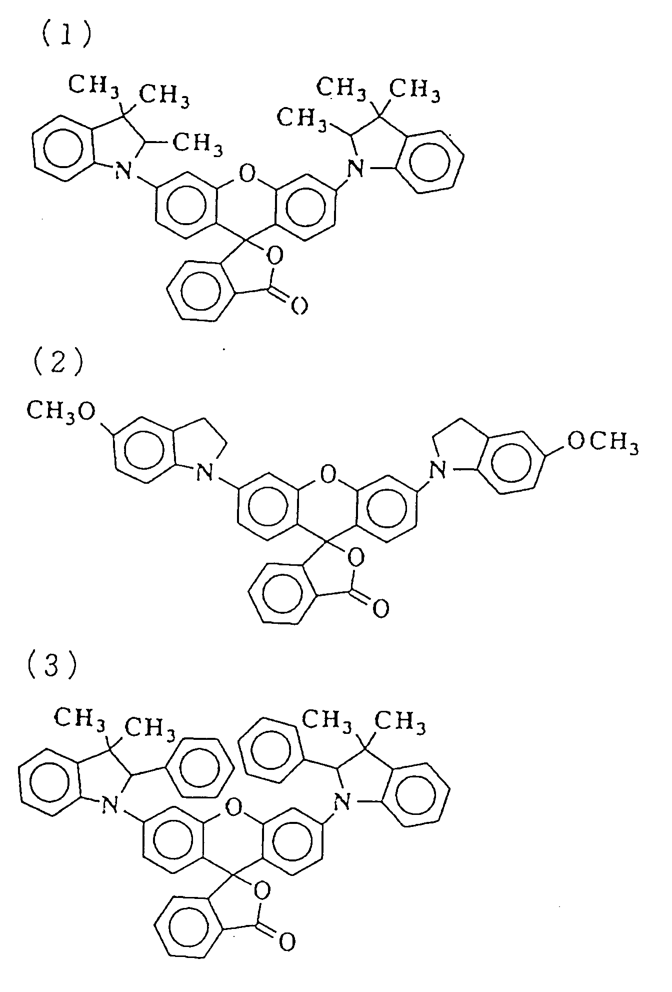

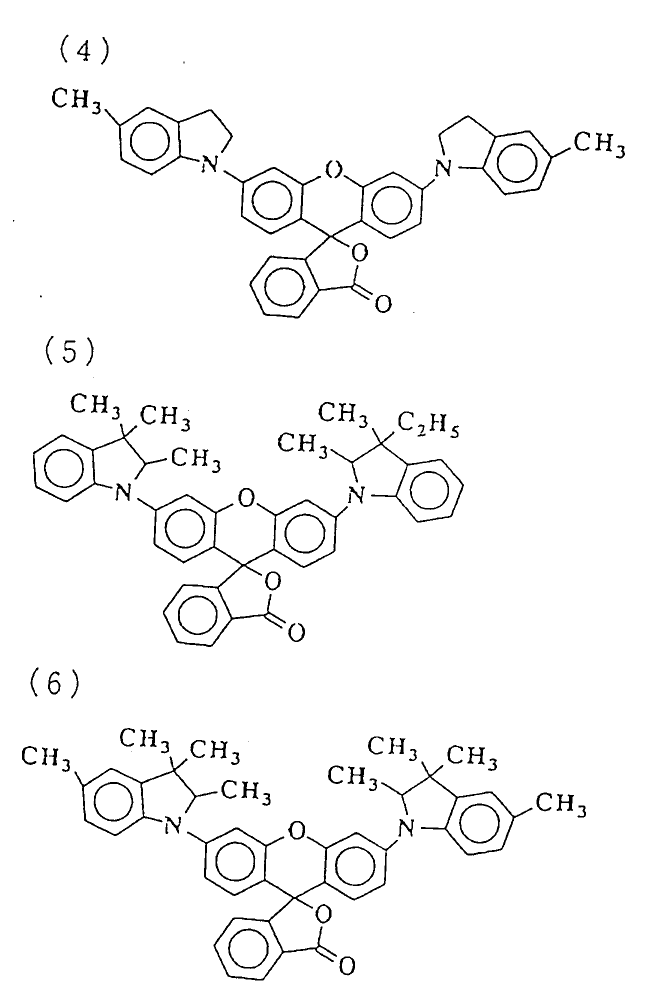

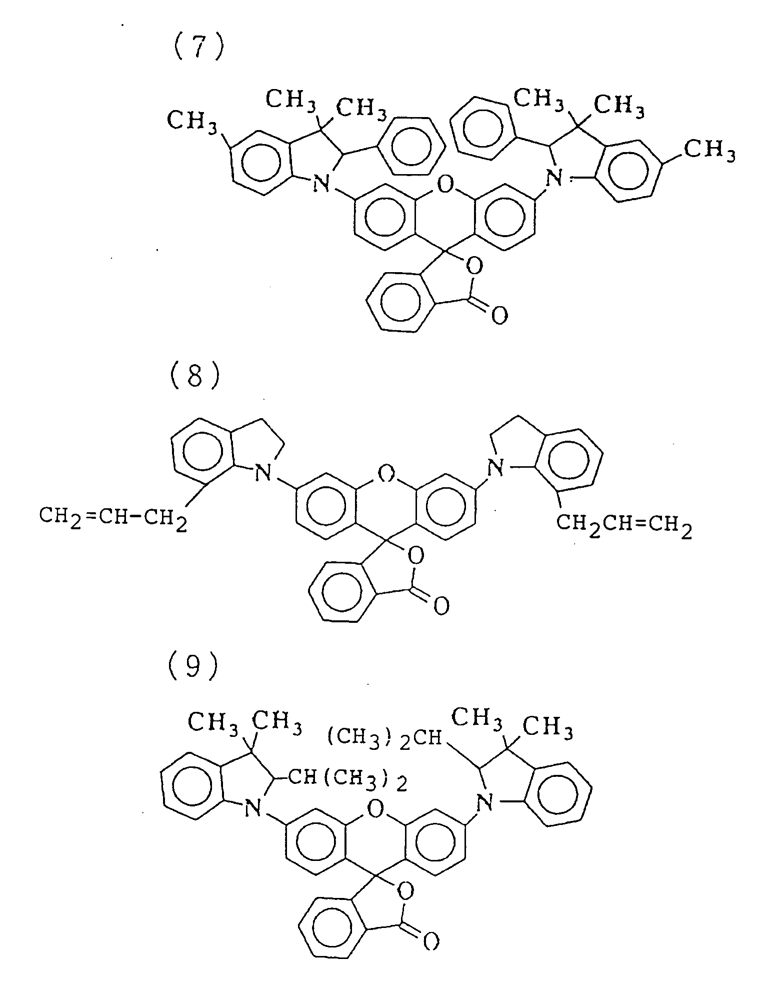

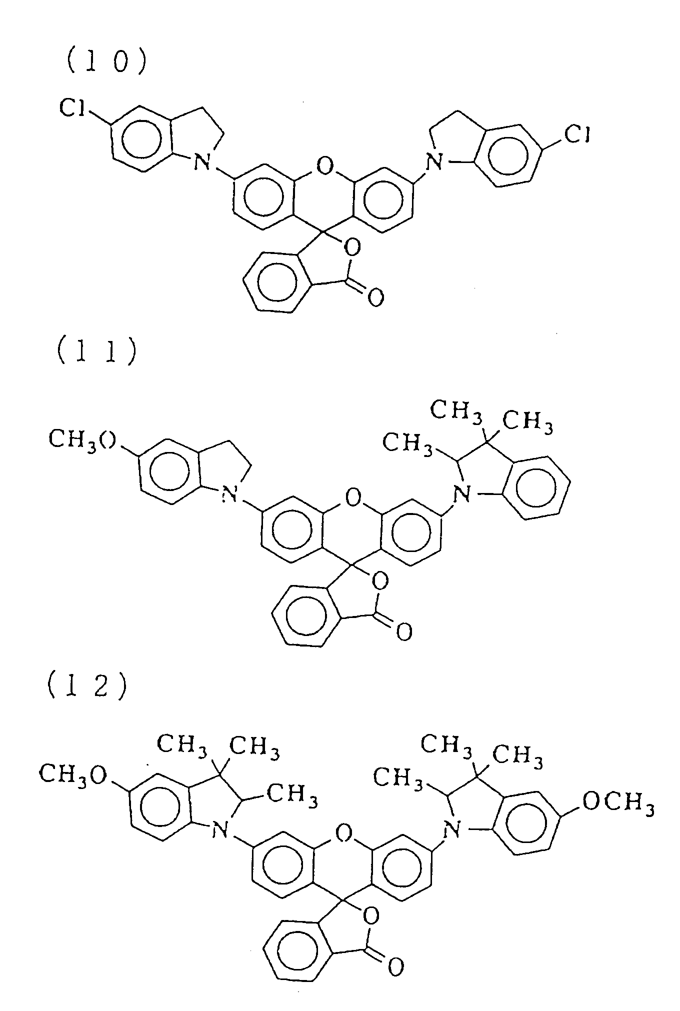

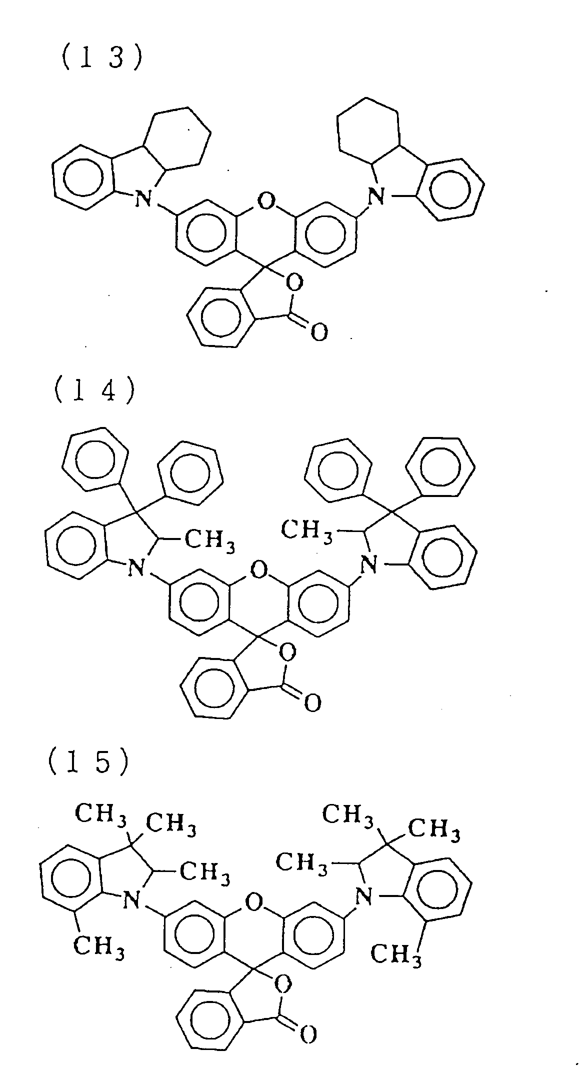

The fluoran compound of the present invention is useful as an electron-donating compound used in a recording material in which an electron-dorating colorless dye and an electron-accepting compound are used, such as a heat sensitive recording material and a pressure sensitive recording material.

The present invention will be further illustrated in the following Examples, but the present invention should not be construed as being limited thereto.

### EXAMPLE 1

### Preparation of the fluoran compound (1)

### (i) Preparation of 2,3,3-trimethylindolenine precursor:

Phenylhydrazine (50g) was mixed with 60g of methylisopropylketone, 50ml of conc. sulfuric acid and 500ml of methanol, and the resulting solution was allowed at a temperature of 70°C, for 8 hours with stirring. After reaction, the reaction mixture was colled to the room temperature and then was neutralized with sodium hydroxide to be extracted with ethylacetate. The ethylacetate phase thus obtained was concentrated with rotary evaporator and then 2,3,3-trimethylindolenine was isolated by a silica gel column chromatography using hexane/ethylacetate developing solvent. (Yield: 60%)

NMR spectral data for the product are shown below:
1.3ppm (s:6H), 2.3ppm (s:3H), 7.15-7.60ppm (m:4H)

### (ii) Preparation of 2,3,3-trimethylindoline intermediate:

Raney nickel catalyst ( Raley-NDNT-90 made by Kawaken fine chemical Corp.) and 150 ml of ethanol were mixed with 2,3,3-trimethylindolenine obtained in the above preparation (i) and left at a temperature of 80°C for 8 hours under hydrogen atomosphere with stirring to proceed the reaction. After reaction, the reaction mixture thus obtained was filtrated and the filtrate was concentrated with a rotary evaporator to isolate 2,3,3-trimethylindoline by a silica gel column chromatography using hexane/ethylacetate developing solvent. (Yield: 80%)

NMR spectral data for the product are shown below:
1.05ppm (s:3H), 1.15-1.20ppm (d:3H), 1.30ppm (s:3H), 3.45-3.55ppm (q:1H), 3.65-3.85ppm (s:1H) disappeared with D₂O, 6.60-7.05ppm (m:4H)

### (iii) Preparation of the fluoran compound (1):

Fluorescein chloride (23g), 21g of zinc chloride and 40ml of sulfolane were added to 25g of 2,3,3-trimethylindoline obtained in the above preparation (ii) and left at a temperature of 160°C for 5 hours with stirring to proceed the reaction. After reaction, water was added to the reaction mixture thus obtained and then extracted with ethylacetate. The ethylacetate phase was concentrated with a rotary evaporator to isolate the fluoran compound (1) with a silica gel column chromathography using hexane/ethylacetate developing solvent. The compound thus obtained has a property of developing to cyan color on a silica gel plate and has a melting point of 157°C and an absorption maximum at 588nm in 95% acetic acid.

### EXAMPLE 2

### Preparation of the fluoran compound (2)

The same procedure as in Example 1 was proceeded except for using 5-methoxyindoline instead of 2,3,3-trimethylindoline used in Example 1. The fluoran compound thus obtained has a property of developing to cyan color on a silica gel plate and has a melting point of 255°C and an absorption maximum at 688nm in 95% acetic acid.

### REFERENCE EXAMPLES 1 TO 4

### (1) Preparation of a developer sheet:

Ten parts of zinc 3,5-bis(α-methylbenzyl)salicylate was added to 20 parts of 1-isopropylphenyl-2-phenylethane and heated at 90°C and dissolved. The resulting solution was added to 50 parts of a 2% aqueous solution of polyvinyl alcohol ("PVA-117" manufactured by Kuraray Co., Ltd. (molecular weight: 75,000)), and 0.1 part of 10% aqueous solution of triethanol amine of dodecylbenzene sulfonate was further added thereto as a surfactant. An emulsion having a particle diameter of 3 µm was prepared by a homogenizer.

Eighty parts of calcium carbonate, 20 parts of zinc oxide, 1 part of sodium hexamethaphosphate and 200 parts of water was dispersed by a kady mill for 10 minutes to prepare a dispersion and then the above-described emulsion was added thereto. Thereafter, 100 parts of 10% aqueous solution of PVA-117 (manufactured by Kuraray Co., Ltd.) and 10 parts by solids content of carboxy-modified SBR latex ("SN-307", manufactured by Sumitomo Naugatuc Co., Ltd.) as a binder were added, and water was added to the mixture to adjust solids content to a concentration of 20%, to prepare coating solution (A).

Ten parts of the developer, 20 parts of silton clay, 60 parts of calcium carbonate, 20 parts of zinc oxide, 1 part of sodium hexamethaphosphate and 200 parts of water were mixed and uniformly dispersed by a sand grinder to prepare a dispersion having an average particle diameter of 3 µm.

To the thus-obtained dispersion, 16 parts of a 10% aqueous solution of PVA-103 (manufactured by Kuraray Co., Ltd.) and 100 parts of a 10% aqueous solution of PVA-117 (manufactured by Kuraray Co., Ltd.) and 10 parts by solids content of carboxy-modified SBR latex ("SN-307", manufactured by Sumitomo naugatuc Co., Ltd.) were added, and thereafter water was added to adjust the solids concentration of the dispersion to 20%, to prepare coating solution (B).

The coating solutions (A) and (B) were mixed in a mixing ratio of A/B=50/50 in terms of the amount of the developer and coated on a base paper (50 g/m²) by an air knife coater to a solids content of 5.0 g/m², and then dried to obtain a developer sheet.

### (2) Preparation of a color former sheet:

Five parts of sodium polyvinylbenzene sulfonate ("VERSA, TL 500", manufactured by national Starch Co., Ltd. (average molecular weight: 500,000)) was dissolved in 95 parts of hot water having a temperature of about 80°C with stirring. After it was dissolved for about 30 minutes, it was cooled. The aqueous solution had a pH of from 2 to 3, and a 20 wt% aqueous solution of sodium hydroxide was added thereto to adjust the pH to 4.0. Then, 100 parts of diisopropyl naphthalene having dissolved therein the color former shown in Table I (electron-donating colorless dye) in an amount of 3.5% was added to 100 parts of a 5% aqueous solution of sodium polyvinylbenzene sulfonate prepared above, emulsified and dispersed to obtain an emulsion having an average particle size (diameter) of 4.5 µm. Six parts of melamine, 11 parts of a 37 wt% aqueous solution of formaldehyde and 30 parts of water were mixed and heated at 60°C with stirring and after 30 minutes, a mixed aqueous solution of transparent melamine, formaldehyde and an initially condensed product of melamine and formaldehyde were obtained. The mixed aqueous solution had a pH of from 6 to 8. Hereinafter, the mixed aqueous solution of melamine, formaldehyde and the initially condensed product of melamine-formaldehyde is referred to as the "initially condensed solution". Forty-seven parts of the initially condensed solution thus obtained was added to the above-described emulsion, mixed and while stirring, the pH thereof was adjusted to 6.0 by the addition of a 3.6 wt% phosphoric acid solution and the solution was heated to 65°C with stirring for 360 minutes. The thus-obtained microcapsule solution was cooled to room temperature, and the pH thereof was adjusted to 9.0 by the addition of a 20 wt% aqueous solution of sodium hydroxide.

Then, 200 parts of a 10 wt% aqueous solution of polyvinyl alcohol (molecular weight: 20,000) and 50 parts of starch particles (size: 15 µm) were added to the microcapsule solution, and water was added thereto to adjust the solids content concentration to 20% to obtain a coating solution containing a microcapsule dispersion.

The coating solution was coated on a base paper (50 g/m²) in a coating amount of 5 g/m² by solids content by an air knife coater, and dried to obtain a color former sheet according to the present invention.

The thus obtained color former sheet and the developer sheet were superposed with the coated layers in contact, and upon pressure of 600 kg/cm₂ cyan colored images were immediately obtained.

**Table 1**

| | | Melting Point (°C) | λmax ( in 95% Acetin Acid) (mm) |
|---|---|---|---|
| 1 | Compound (5) | 150 | 640 |
| 2 | Compound (23) | semi-solid | 642 |
| 3 | Compound (29) | 170 | 656 |
| 4 | Compound (24) | semi-solid | 645 |

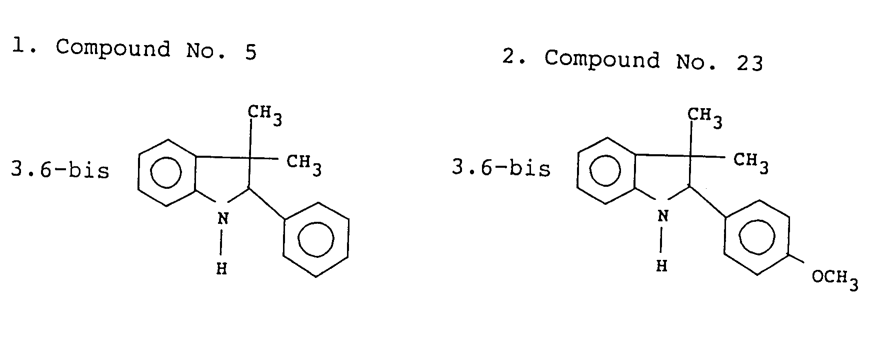

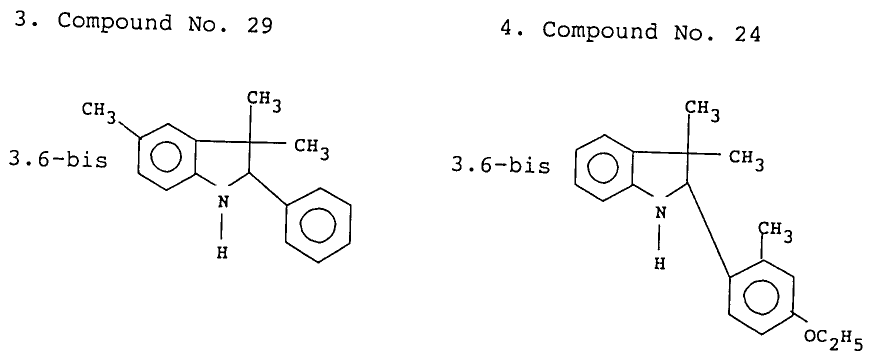

While the invention has been described in detail and with reference to specific embodiments therof, it will be apparent to one skilled in the art that various changes and modifications can be made therein without departing from the spirit and scope thereof.

## Claims

1. A fluoran compound represented by formula (I): 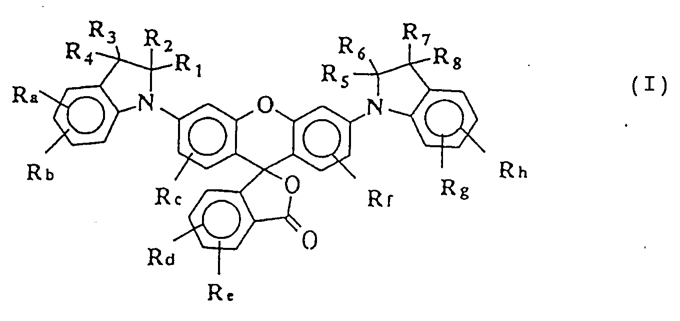 wherein R₁, R₂, R₃, R₄, R₅, R₆, R₇ and R₈ each independently represent a hydrogen atom, a substituted or unsubstituted alkyl group, a substituted or unsubstituted aryl group, a substituted or unsubstituted alkoxy group or halogen atom, and R₁ to R₄, and/or R₅ to R₈ may be combined to form a ring, and wherein Rₐ, R_{b}, R_{c}, R_{d}, Rₑ, R_{f}, R_{g} and Rₕ each represent a hydrogen atom, a substituted or unsubstituted alkyl group, a substituted or unsubstituted aryl group, a substituted or unsubstituted alkoxy group, a substituted or unsubstituted alkenyl group, a halogen atom, a substituted amino group, a substituted carbamoyl group, an acyl group, a thioalkoxy group, an aryloxy group or a nitro group.

2. A fluoran compound as claimed in claim 1, wherein R₁ to R₈ each independently represent a methyl group, ethyl group, propyl group, isopropyl group, butyl group, isobutyl group, pentyl group, isopentyl group, phenyl group, benzyl group, methoxy group, ethoxy group, or propoxy group, or a hydrogen, chlorine, bromine or fluorine atom.

3. A fluoran compound as claimed in claim 1 or 2, wherein Rₐ to Rₕ each independently represent a hydrogen atom, a methyl group, ethyl group, propyl group, isopropyl group, butyl group, isobutyl group, pentyl group, isopentyl group, phenyl group, benzyl group, methoxy group, ethoxy group, propoxy group, vinyl group, allyl group, propenyl group, chlorine atom, bromine atom, fluorine atom, diethylamino group, ethyl amino group, anilino group, benzoylamino group, thiomethoxy group, thiophenoxy group, phenoxy group, p-methoxyphenoxy group or phenoxyphenoxy group.

4. A fluoran compound as claimed in claim 2 or 3, wherein one or more of the groups represented by R₁ to R₈ and Rₐ to Rₕ are substituted with an alkyl group, carboxyl group, hydroxy group, alkoxy group, amino group, phenyl group or benzyl group, or a hydrogen atom.

5. A fluoran compound as claimed in claim 2 or 3, wherein one or more of the groups R₁ to R₈ and Rₐ to Rₕ are substituted with 2-ethylhexyl, n-octyl, dodecyl, pentadecyl, t-decyl, p-t-amylphenyl, p-cumylphenyl, p-phenoxyphenyl, p-dodecylphenyl, 2,4-dibutoxyphenyl or 2,4-di-t-amylphenyl.

6. A fluoran compound as claimed in any preceding claim, wherein the groups of R₁ and R₂, and R₃ and R₄ are the same as those of R₅ and R₆, and R₇ and R₈, respectively, and the groups of Rₐ and R_{b} are the same as those of R_{g} and Rₕ, and wherein the substituted position of Rₐ and R_{b} is the same as that of R_{g} and Rₕ, in each indoline ring.

7. A process for producing a fluoran compound as claimed in claim 1, comprising reacting fluorescein chloride and/or fluorescein bromide in the presence of a solvent, and zinc chloride and/or aluminum chloride, with an indoline of the following formula (II): 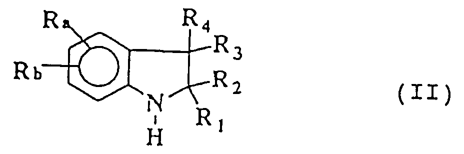 wherein R₁, R₂, R₃ and R₄ each represent a hydrogen atom, a substituted or unsubstituted alkyl group, a substituted or unsubstituted aryl group a substituted or unsubstituted alkoxy group or halogen atom, wherein R₁ to R₄ and/or Rₐ to R_{b} may be combined to form a 5- to 12-membered ring comprising a non-metallic atom, and Rₐ and R_{b} are each and defined in claim 1.

8. A process for producing a fluoran compound as claimed in claim 1, comprising reacting a 2-hydroxybenzoylbenzoic acid represented by the following formula (III): 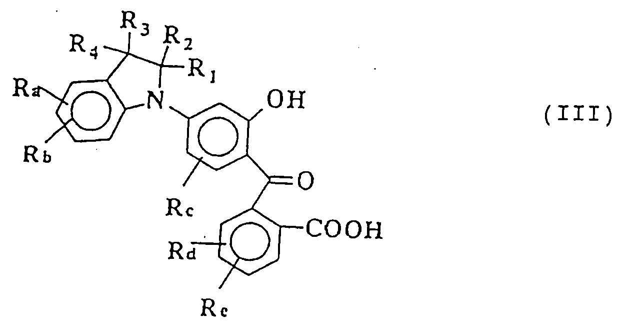 wherein R₁, R₂, R₃ and R₄ each represent a hydrogen atom, a substituted or unsubstituted alkyl group, a substituted or unsubstituted aryl group, a substituted or unsubstituted alkoxy group or halogen atom, wherein R₁ to R₄ and/or Rₐ to R_{b} may be combined to form a ring, and Rₐ, R_{b}, R_{c}, R_{d} and Rₑ each represent a hydrogen atom, a substituted or unsubstituted alkyl group, a substituted or unsubstituted aryl
group, a substituted or unsubstituted alkoxy group, a substituted or unsubstituted alkenyl group, halogen atom, a substituted amino group, a substituted carbamoyl group, an acyl group, a thioalkoxy group, an aryloxy group or nitro group, with an indoline represented by the following formula (IV): 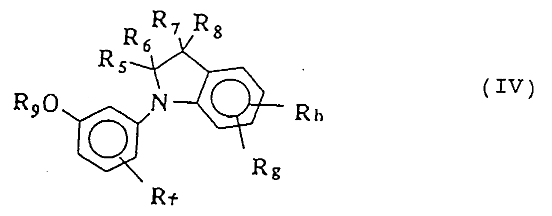 wherein R₅, R₆, R₇ and R₈ each represent a hydrogen atom, a substituted or unsubstituted alkyl group, a substituted or unsubstituted aryl group, a substituted or unsubstituted alkoxy group or halogen atom, wherein R₅ to R₈ and/or R_{g} to Rₕ may be combined to form a ring, and R_{f}, R_{g} and Rₕ each represent a hydrogen atom, a substituted or unsubstituted alkyl group, a substituted or unsubstituted aryl group, a substituted or unsubstituted alkoxy group, an alkenyl group, a halogen atom, a substituted amino group, a substituted carbamoyl group, an acyl group, a thioalkoxy group, an aryloxy group or nitro group, and R₉ represents a lower alkyl group.

9. A heat-sensitive or pressure-sensitive recording material incorporating a fluorine compound as claimed in any preceding claim.
